# EUROPEAN PATENT APPLICATION

(11) **EP 4 467 965 A1**
(43) Date of publication of application: **27.11.2024**
(21) Application number: 23174863.3
(22) Date of filing: 23.05.2023
(51) Int. Cl.: G01N 21/25, G01N 21/3563, G01N 23/00, B07C 5/34, G01N 21/359, G01N 21/84, G01J 3/46

(54) **TEXTILE IDENTIFICATION SYSTEM AND METHOD OF IDENTIFYING TEXTILES**

(71) Applicant: Universal Textile Sorting ApS, 8840 Rødkærsbro (DK)
(72) Inventor: Balle, Morten, 8840 Rødkærsbro (DK); Larsen, Henrik Lau, 8840 Rødkærsbro (DK); Enevoldsen, Andreas Lehmann, 8840 Rødkærsbro (DK); Lindgaard, Morten, 8840 Rødkærsbro (DK)
(74) Representative: Patrade A/S

(57) **Abstract**

The present invention relates to a textile identification system for identifying textile subjects, wherein said identification system comprises: a conveyor for moving an associated textile subject in a first direction, a visible light emitter for emitting light within a visible wavelength spectrum, a colour image sensor for measuring light within a visible wavelength spectrum, an infrared light emitter for emitting light within an infrared wavelength spectrum, an infrared light sensor for measuring light within an infrared wavelength spectrum, a radiation emitter for emitting radiation within an X-ray spectrum or gamma ray spectrum, a radiation sensor for measuring radiation within an X-ray spectrum or a gamma spectrum, a database system adapted to at least retrieve, store and process the data measured by the sensors. Furthermore, the invention relates to a method for identification of a textile subject and moreover to a computer program and a computer-implemented method of classifying textile subjects.

## Description

### FIELD OF THE INVENTION

The present invention relates to a textile identification system for identifying textile subjects, wherein said identification system comprises: a conveyor for moving an associated textile subject in a first direction, a visible light emitter for emitting light within a visible wavelength spectrum, a colour image sensor for measuring light within a visible wavelength spectrum, an infrared light emitter for emitting light within an infrared wavelength spectrum, an infrared light sensor for measuring light within an infrared wavelength spectrum, a radiation emitter for emitting radiation within an X-ray spectrum or gamma ray spectrum, a radiation sensor for measuring radiation within an X-ray spectrum or a gamma spectrum, a database system adapted to at least retrieve, store and process the data measured by the sensors. Furthermore, the invention relates to a method for identification of a textile subject and moreover to a computer program and a computer-implemented method of classifying textile subjects.

### BACKGROUND OF THE INVENTION

The world calls for greener and more sustainable solutions, and a huge area where the sustainability can be optimized and utilized is the textile reutilisation area, especially the mechanical recycling and/or reuse of textile. Therefore, a system and method for optimizing the possibilities of reusing textile are highly requested.

Within the area of reutilisation of textile, and especially within the area of reusing textile fibers, it might seem easy and straight forward to provide and perform such recycling and/or reuse processes. However, the need for ideal preliminary processes before textiles of all kinds can be mechanically processed into reusable fibers is both essential but also extremely challenging.

Before textile can be recycled and/or reused properly, proper identification of one or more characteristics of all the diverse and various textiles is necessary, such that the textile selected for recycling and/or reuse can be identified, classified and/or sorted properly before the subsequent recycling processes.

Today, solutions for preliminary processes of identifying, classifying and/or sorting textile for recycling and/or reuse purposes are available, however, none of the known systems and methods provide an optimal solution.

It is a known problem that even though a huge amount of textiles is collected for the purpose of reusing the textile fibers, the majority of the collected textile never ends in a fiber reuse process, simply because the characteristics of the textile cannot be identified properly. This means that the textile is not sorted correctly, and in the end, the final process of making a textile into reusable fibers cannot be performed, since the fibers will be destroyed, if they are not opened correctly in the reuse process.

The sad consequence of this is that the textile meant for recycling and/or reuse is either incinerated or landfilled, which is a waste of resources and damages the environment instead of providing reusable and more sustainable textile fibers.

Hence, an improved system and method, preferably an efficient and reliable system and method, suitable for identifying, classifying and/or sorting textile would be advantageous, and in particular a more optimal, reliable, efficient, precise, economically and sustainable system and method would be advantageous.

### OBJECT OF THE INVENTION

It is an object of the present invention to provide an alternative to the prior art.

In particular, it may be seen as an object of the present invention to provide a system and a method that solve the above-mentioned problems by providing optimal preliminary processes for textiles to be mechanically recycled and/or reused.

### DESCRIPTION OF THE INVENTION

Thus, the above-described object and several other objects are intended to be obtained in a first aspect of the invention by providing a textile identification system for identifying textile subjects, wherein said identification system comprises:
- a conveyor for moving an associated textile subject in a first direction,
- a visible light emitter for emitting light within a visible wavelength spectrum,
- a colour image sensor for measuring light within a visible wavelength spectrum, preferably a RGB sensor,
- an infrared light emitter for emitting light within an infrared wavelength spectrum, preferably within a near infrared wavelength spectrum,
- an infrared light sensor for measuring light within an infrared wavelength spectrum, preferably within a near infrared spectrum,
- a radiation emitter for emitting radiation within an X-ray spectrum or gamma ray spectrum,
- a radiation sensor for measuring radiation within an X-ray spectrum or a gamma spectrum,
- a database system adapted to at least retrieve, store and process the data measured by at least:
   - the colour image sensor,
   - the infrared light sensor, and
   - the X-ray or the gamma ray sensor.

The invention is particularly, but not exclusively, advantageous for providing a system wherein information of textile for being recycled and/or reused is uniquely provided, not only by a single sensor, but by at least three separate sensors, each providing essential information of the textile subject. Furthermore, and most exceptional - the interplay between these three specific sensors has until now not been seen before, and this interplay has turned out to provide unique and exclusive information within textile identification, classification, and sorting. This arrangement provides a system suitable for identifying any necessary information of a textile needed to optimize the subsequent mechanical recycling.

The system thereby consists of a combination of three specific sensor types for the classification of textile subjects: an X-ray sensor, an infrared sensor and a colour image sensor. The textile subject is transported on a conveyor and passes underneath the sensors, which each gather "images" of the textiles at different wavelengths of the electromagnetic wave spectrum.

As an example, this system with the three specific sensors provides the possibility to infer with a certain probability that the textile is a pair of denim jeans, while none of the sensors can do this by themselves.

Having this unique combination of sensors allows for identification, classification and sorting of the textiles which is optimized for improving the fibre yield and quality in subsequent recycling processes.

It should be understood that the particular order of the sensors is not relevant within the context of the invention, only the presence of the sensors is essential.

It should moreover be understood that the system can be combined with further sensors and/or other further devices, and the invention is not limited to necessarily only comprise the three sensors, as long as the three sensors are present in the system, more sensors of the same type and/or other sensor-types and/or other devices might be included in the system.

Within the context of the invention, the "textile subject" can be any type of textile material, e.g.: every type of clothing/clothes/garments/wear/apparel, household textiles, outdoor gear, non-woven materials, cleaning textiles, workwear, shoes etc. The type of textile should therefore not be seen as limiting to the invention.

By exposing the textile subject to the three different types of electromagnetic radiation and measuring the reflectance and/or absorption using specialized sensors, the following sensor outputs can be provided:
- The colour image sensor, being focused on visible light.
   The sensor allows a colour detection by measure of the colour distribution over the textile.
   Furthermore, the sensor can extract certain characteristics from the images such as reflective material, polymer prints, accessories (buttons, zippers, other attachments, etc.) or fabric type (weave, knit, terry cloth, denim, etc.), pattern, and/or preferably also providing information about the characteristic of the material composition (at a molecule level).
   Potentially, a machine learning algorithm can be used to optimize the information extraction.
- The infrared sensor, being focused on the infrared spectrum, preferably using reflectance spectroscopy / diffused reflectance spectroscopy.
   The sensor allows for material detection by measuring the reflected light spectrum.
   The sensor can detect the material composition of the textile subject.
   Furthermore, the infrared sensor can detect moisture or specific contaminant chemicals such as oil on workwear, mould on old textiles or dirt.
   Potentially, a machine learning powered classification algorithm might be used in a multi-stage classification scheme.
- The X-ray or gamma ray sensor, being focused on the X-ray or gamma ray spectrum, preferably using absorption spectroscopy such as X-ray or gamma ray absorption spectroscopy.
   A radiation source emits X-rays or gamma rays to pass through the textile subject. The rays are measured by a ray sensor underneath the textile subject.
   The ray sensor allows for the detection of density and can thus also be used to detect hard material parts such as accessories.
   Furthermore, the sensor can be used to infer the mass of the textile subject. Potentially, a machine learning algorithm might be trained to detect common accessories such as buttons, zippers and pens or tools.

The database system is suitable for interpreting the sensor outputs and performing classification using a probabilistic machine learning model.

The system thereby also provides optimal conditions for inferring information using a probabilistic model.

By using the different sensor outputs, several different characteristics of the textile subject can be derived with varying certainties. Using a probabilistic machine learning model (Bayesian network), the probability of the textile subject having certain characteristics can be estimated.

In the context of the present invention, a "color image sensor " may be understood as a sensor that can measure visible light along a line (line scan) or over an area (area scan) to provide a digital color image in an RGB format, or similar digital color image representations.

An example of a color image sensor, which is preferred within the invention, is an RGB sensor, being a color sensor employed to recognize/detect the color of a material in RGB (red, green, blue) format and/or a CMOS sensor, employed to obtain color images of the textile subject in RGB (red, green, blue) format to recognize/detect the color of a material.

Furthermore, this system provides the opportunity to use all, or at least as much possible, of the textiles intended for recycling and/or reuse, since all the necessary characteristics of the textile are correctly identified, also a correct classification of the textile can be made and, in the end, an optimal and reliable sortation of the textile is performed, such that the sorted textile is ready to be recycled and/or reused by opening up the fibers without destroying them.

This means that a sorting system is utilized in all its potential, since more or less all textiles can be processed optimally and thereby can be mechanically recycled and/or reused in the most sustainable way. By this means, an extreme green, sustainable, and also economically viable system is provided.

The process of recycling the fibers, e.g. by opening up the fibers in a mechanical recycling process, should not be seen as a part of the invention, but a process suitable to be performed after the textile subject has been sorted by the system. However, the invention can also be used to sort textiles for other recycling processes, such as chemical recycling.

In one embodiment of the invention, the database system is adapted to identify at least one predetermined characteristic of the associated textile subject.

The embodiment is particularly, but not exclusively, advantageous for providing a system wherein characteristics of textile can be identified reliably.

In one embodiment of the invention, the identification system further comprises an algorithm, said algorithm is adapted to provide a classification of the associated textile subject based on the at least one predetermined characteristic.

The embodiment is particularly, but not exclusively, advantageous for providing an optimized system for classifying the textiles into predetermined classification groups. The data is processed through at least one algorithm to get usable information out of the data measured by the sensors - that is, to go from raw data to information that classification can be based on.

In one embodiment of the invention, the identification system is adapted to provide a command for sortation of the associated textile subject based on the classification.

The embodiment is particularly, but not exclusively, advantageous for providing an optimized system for sorting the textiles into predetermined sortation groups.

In one embodiment of the invention, the algorithm is a machine learning algorithm adapted to be trained by the collected data from the database.

The embodiment is particularly, but not exclusively, advantageous for providing a system wherein the system improves itself every time a textile subject runs through the system. By employing a machine learning algorithm, the system will in time identifying characteristics of the textile subject, classifying the textile subject and/or sorting the textile subject with an accuracy continuously increasing up to potentially being about 100% correct and reliable.

In one embodiment of the invention, the radiation sensor is positioned underneath the conveyor, and wherein the conveyor or part of the conveyor above the radiation sensor is made of material suitable for not blocking X-rays.

The embodiment is particularly, but not exclusively, advantageous for allowing extraction of information about a textile subject through conveyor (belt) material, and still provide optimal and usable information from the X-ray or gamma ray sensor, meaning that even though the measurement of the sensor is achieved through other material then the textile subject, the measurement can be provided only about the textile such that the measured conveyor data does not influence the textile data. The material of the conveyor (belt) provides a "constant noise" in the data / pictures collected by the sensor, and the constantness makes is easier to remove the "noise" and thereby get an optimal picture of the textile via the radiation sensor, even though the measurement is made though the material of the conveyor.

In one embodiment of the invention, the conveyor comprises a gap, and wherein the radiation sensor is positioned underneath the gap of the conveyor.

The embodiment is particularly, but not exclusively, advantageous for allowing extraction of information about a textile subject transported in a conveyor, but without having to measure information through conveyor (belt) material, since the measurement is made at the gap, meaning there is no disturbing data from the measurement.

In one embodiment of the invention, the position of the textile subject on the conveyor is measured by at least one of the sensors, and wherein a robot, preferably a robot arm, is adapted to collect the subject for sortation.

The embodiment is particularly, but not exclusively, advantageous for providing a reliable sortation of the textile subject after being identified and classified.

In one embodiment of the invention, the system further comprises a sortation air-system, such as a fan system and/or air nozzles with pressurized air, being adapted for sortation of the textile subject.

The embodiment is particularly, but not exclusively, advantageous for providing a reliable sortation of the textile subject after being identified and classified.

In one embodiment of the invention, the system comprises multiple reversible overlapping sorting conveyors adapted to receive the textile subject from the at least one main conveyor and transport the textile subject for sortation.

The embodiment is particularly, but not exclusively, advantageous for providing a reliable sortation of the textile subject after being identified and classified.

In one embodiment of the invention, the position of the textile subject is being manually sorted.

The embodiment is particularly, but not exclusively, advantageous for providing a reliable sortation of the textile subject after being identified and classified.

In one embodiment of the invention, the system further comprises a shielding for shielding from the radiation of the radiation emitter.

The embodiment is particularly, but not exclusively, advantageous for providing a system secured from X-ray and gamma radiation.

In one embodiment of the invention,
- the infrared light emitter is a halogen lamp or a LED bulb and/or
- the infrared light sensor is a near infrared (NIR) sensor or a mid-infrared (MIR) sensor.

In a second aspect, the invention further relates to a method for identification of a textile subject, preferably in an identification system according the first aspect of the invention, wherein said method comprises the steps of:
- transporting a textile subject in a first direction, preferably on a conveyor,
- emitting light at the textile subject by at least one first light emitter, said first light emitter emitting light within the visible light spectrum,
- measuring colour distributed over the textile subject by at least one colour image sensor, preferable a RGB sensor,
- emitting infrared light on the subject by at least one second light emitter,
- measuring reflected light by at least one infrared light sensor,
- emitting x-ray or gamma-ray radiation through the textile by a radiation emitter,
- measuring radiation by at least one radiation sensor,
- collecting data measured by the colour image sensor, the infrared sensor and the radiation sensor,
- identifying at least one characteristics of the textile subject, preferably identify one or more of:
   - the colour,
   - the material composition, and
   - the presence of hard material, such as buttons and zippers, and
- classifying the subject, preferably into predetermined classification groups,
wherein the steps of the method can be executed in any order and/or executed simultaneously.

The second aspect of the invention is particularly, but not exclusively, advantageous for providing a method for identification of a textile subject, where information of textile for being recycled and reused is uniquely provided, not only by a single sensor but by at least three separate sensors, each providing essential information about the textile. Furthermore, and most exceptional - the interplay between these three specific sensors has until now not been seen before, and this interplay has turned out to provide unique and exclusive information within textile identification, classification, and sorting. This method provides a process suitable for identifying any necessary information of a textile.

This method provides the possibility to infer with a certain probability that the textile is a pair of denim jeans, while none of the sensors can do this by themselves.

Furthermore, this method provides the opportunity to use all, or at least as much possible, of the textiles intended for recycling and/or reuse, since all the necessary characteristics of the textile are correctly identified, also a correct classification of the textile is made and, in the end, an optimal and reliable sortation of the textile is performed, such that the sorted textile is ready to be recycled and/or reused by opening up the fibers mechanically without destroying them, or utilizing other recycling methods such as chemical recycling.

The process of recycling the fibers, be e.g. opening up the fibers, should not be seen as a part of the invention, but a process suitable to be performed after the textile subject has been sorted by the system.

In one embodiment of the invention, the method further comprises the step of
- removing the "background noise" of the conveyor in the measured data of the radiation sensor.

The embodiment is particularly, but not exclusively, advantageous for achieving measurement only of the textile, wherein the conveyor does not influence the data. The material of the conveyor (belt) provides a "constant noise" in the data / pictures collected by the sensor, and the constancy makes is easier to remove the noise and thereby get an optimal picture of the textile via the radiation sensor even though the measurement is made though the material of the conveyor.

Within the invention both for the system and the method, there might be provided different conveyors for the different sensors, such that the removal of the background noise is made most optimal for the different sensor types.

In one embodiment of the invention, the method further comprises the step of:
- storing the measured and collected data in a database.

The embodiment is particularly, but not exclusively, advantageous for potentially using all the stored information to advance the system.

In one embodiment of the invention, the method further comprises the step of:
- providing at least one algorithm.

The embodiment is particularly, but not exclusively, advantageous for a method wherein characteristics of textile can be identified reliably.

The data is processed, preferably, but not necessarily, through various algorithms to get usable information out of the data measured by the sensors - that is, to go from raw data to information that identification can be based on.

In one embodiment of the invention, the method further comprises the step of:
- using the stored data in the database for machine learning by training the at least one algorithm,
   said algorithm preferably being trained for at least one of:
   - optimizing the identification of the characteristics of the textile subject,
   - optimizing the classification of the subject, and/or
   - optimizing the sortation of the subject.

The embodiment is particularly, but not exclusively, advantageous for providing a possibility of using machine learning at (at least) three levels, such as:
- The optimization of the identification can be used to see specific characteristics (e.g. recognize a button on an X-ray).
   By using the different sensor outputs, several different characteristics of the textile subject can be derived with varying certainties. Using a probabilistic machine learning model (Bayesian network), the probability of the textile subject having certain characteristics can be estimated given the measured data.
- The optimization of the classification can be used to classify on the basis of identified characteristics (e.g. recognize that light blue cotton with a button and a zipper with 89% probability is a pair of jeans - simplified example).
- The optimization of the sortation can be used to allow most possible textile to be recycled and/or reused.

In one embodiment of the invention, the classification of the subject is based on the at least one algorithm.

The embodiment is particularly, but not exclusively, advantageous for providing an improved method for classification.

In one embodiment of the invention, the method further comprises the step of:
- choosing predetermined sortation groups,
- sorting the subject based on the one or more predetermined sortation groups.

In one embodiment of the invention, the sortation of the subject is based on the at least one algorithm.

The embodiments are particularly, but not exclusively, advantageous for providing a method wherein the sortation of textile subjects is made more exact and precise than the current state of art, taking the potential of reusing textile to an until now unseen level.

In one embodiment of the invention, the method further comprises the step of:
- using the machine learning algorithm for at least one of:
   - identification of characteristics of the textile subject,
   - classification of the subject into predetermined classification groups, and/or
   - sortation of the subject into predetermined sortation groups.

The embodiment is particularly, but not exclusively, advantageous for providing an improved method for identification, classification and/or sortation, and further provide a method that will keep improving itself every time the method has been performed.

In one embodiment of the invention, the characteristics of the textile subject identified from the measurements of the one or more sensors is one or more of:
- colour distribution, such as colour gradients
- fabric type, such as weave, knit, terry cloth and/or denim,
- material composition,
- density,
- mass,
- condition, such as cleanness, moisture content and/or mould content,
- presence of reflective material,
- presence of pattern, such as stripes and/or squares,
- presence of polymer prints,
- presence of hard accessories such as buttons, zippers, pens and/or tools,
- presence of contaminant chemicals such as oil,
- presence of multi-layer textile, such as jackets, pillows and duvets,
- type of textile subject, such as trousers, jeans, a shirt, a bag or a shoe, or
- the subject is not a textile subject.

In one embodiment of the invention, within the system or within the method:
- the visible light spectrum is within 300-1000 nm., and/or
- the infrared spectrum is within 1300-4000 nm., and/or
- the X-ray or gammy ray spectrum is within 0.01-10 nm.

In a third aspect, the invention further relates to computer program comprising instructions which, when the program is executed by a computer, cause the computer to carry out at least the identifying and classification, preferably also the sortation.

In a fourth aspect, the invention further relates to computer-implemented method of classifying textile subjects, comprising:
- receiving an input dataset, according to the measurement steps, the input dataset comprising at least one identified characteristic of the textile subject; and
- producing an output dataset comprising a classification of the textile subject.

The first, second, third and fourth aspect of the present invention may each be combined with any of the other aspects. These and other aspects of the invention will be apparent from and elucidated with reference to the embodiments described hereinafter.

Embodiments from the system/product should be seen as applicable to the method, and embodiments from the method should be seen as applicable for the product/system.

### DESCRIPTION OF THE DRAWING

Figure 1 illustrates a simplified version of the textile identification system, the system being suitable for performing a method for identification of a textile subject.
Figure 2 illustrates a gap in the conveyor.

### DETAILED DESCRIPTION OF THE INVENTION

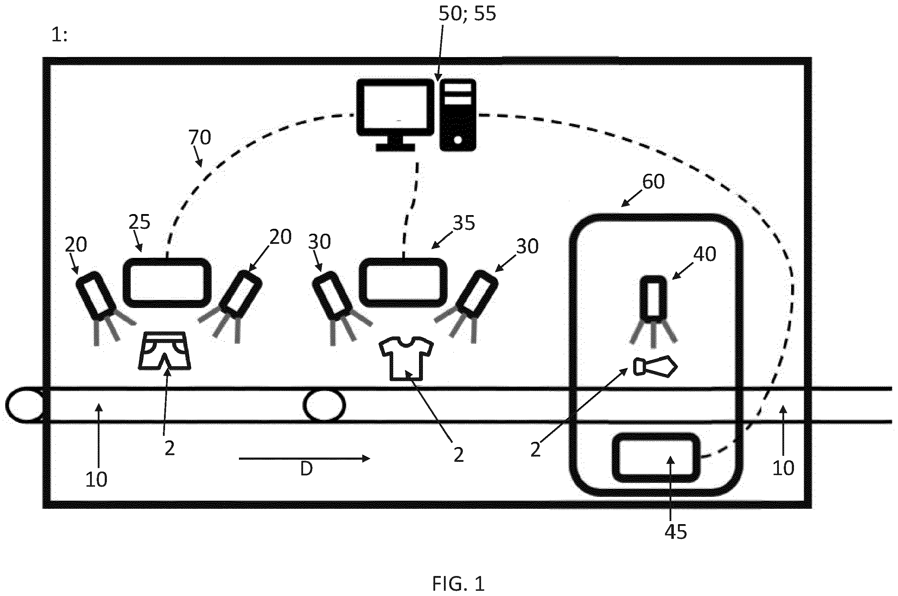

FIG. 1 illustrates a simplified version of the textile identification system 1 for identifying textile subjects 2, wherein said identification system comprises:
- A conveyor 10 for moving an associated textile subject 2 in a first direction D. In FIG. 1, only one main conveyor 10 is illustrated, however, the system may comprise a plurality of main conveyors. Also, the system might comprise sortation conveyors (not shown) at the end of the at least one main conveyor, suitable for sorting the classified textile subject.
- A visible light emitter 20 for emitting light within a visible wavelength spectrum. In FIG. 1, two visible light emitters 20 are illustrated. Within the invention there may also be only one visible light emitter or there may be more than two visible light emitters. The visible light spectrum is preferably within 300-1000 nm.
- A colour image sensor 25 for measuring light within a visible wavelength spectrum, preferably a RGB sensor. In FIG. 1, one colour image sensor 25 is illustrated. Within the invention there may also be a plurality of colour image sensors.
- An infrared light emitter 30 for emitting light within an infrared wavelength spectrum, preferably within a near infrared wavelength spectrum. In FIG. 1, two infrared light emitters 30 are illustrated. Within the invention there may also be only one infrared light emitter or there may be more than two infrared emitters. The infrared light emitter(s) might be a halogen lamp or a LED pulp. The infrared spectrum is preferably within 1300-4000 nm.
- An infrared light sensor 35 for measuring light within an infrared wavelength spectrum, preferably within a near infrared spectrum. In FIG. 1, one infrared light sensor is illustrated. Within the invention there may also be a plurality of infrared light sensors. The infrared light sensor preferably being a near infrared (NIR) sensor and/or a mid-infrared (MIR) sensor.
- A radiation emitter 40 for emitting radiation within an X-ray spectrum or gamma ray spectrum. In FIG. 1, one radiation emitter 40 is illustrated. Within the invention there may also be only one radiation emitter or there may be a plurality of radiation emitters. The X-ray or gammy ray spectrum is preferably within 0.01-10 nm.
- A radiation sensor 45 for measuring radiation within an X-ray spectrum or a gamma spectrum. In FIG. 1, one radiation sensor 45 is illustrated. Within the invention there may also be a plurality of radiation sensors.
- A database system 50 adapted to at least retrieve, store, and process the data measured by at least:
   - the colour image sensor 25,
   - the infrared light sensor 35, and
   - the X-ray or the gamma ray sensor 45.

In FIG. 1, the radiation sensor 45 is positioned underneath the conveyor 10 and the conveyor or part of the conveyor above the radiation sensor is made of material suitable for not blocking X-rays or gamma rays.

It should be understood that the sensor(s) might be positioned underneath the conveyor and the emitter(s) positioned above the conveyor. The positions in FIG. 1 should not be seen as limiting for the invention, but should be understood as a preferred embodiment.

The system further comprises a shielding 60 for shielding from the radiation of the radiation emitter.

The system in FIG. 1 further comprises an algorithm 55, said algorithm is adapted to provide a classification of the associated textile subject 2 based on the at least one predetermined characteristic.

In FIG. 1, the dotted line 70 represents flow of data from the sensors to the database 50. The data collected in the database 50 and connected with an algorithm 55 provides an exclusive solution. While some of the information/characteristics of the textile subject are uniquely provided by a single sensor, other information/characteristic types can only be inferred using all three sensors 25, 35, 45 and using the information from the sensors in the algorithm 55, such as using a probabilistic model. With the system 1, it is now possible with a certain probability to identify characteristic(s) of a textile subject 2 and from those characteristics classify the textile subject as e.g. a pair of denim jeans, while none of the sensors can do this by themselves.

The system illustrated in FIG. 1 is suitable for performing a method for identification of a textile subject 2, wherein said method comprises the steps of:
- Transporting a textile subject in a first direction D, preferably on a conveyor 10,
- Emitting light at the textile subject by at least one visible light emitter, said first light emitter emitting light within the visible light spectrum,
- Measuring colour distributed over the textile subject by at least one colour image sensor, preferable a RGB sensor,
- Emitting infrared light on the subject by at least one second light emitter,
- Measuring reflected light by at least one infrared light sensor,
- Emitting X-ray or gamma-ray radiation through the textile by a radiation emitter,
- Measuring radiation by at least one radiation sensor,
- Collecting data measured by the colour image sensor, the infrared sensor and the radiation sensor,
- Identifying at least one or more of the characteristics of the textile subject,
   - the colour,
   - the material composition, and
   - the presence of hard material, such as buttons and zippers, and
   - Classifying the subject, preferably into predetermined classification groups,
wherein the steps of the method can be executed in any order and/or executed simultaneously. Also, the method may comprise more steps.

Furthermore, FIG. 1 is indirectly illustrating a computer program, comprising instructions which, when the program is executed by a computer, cause the computer to carry out at least the identifying and classification step of the method, preferably also the sortation step.

Furthermore, is FIG. 1 indirectly illustrating a computer-implemented method of classifying textile subjects, comprising:
- receiving an input dataset according to the measurement steps, the input dataset comprising at least one identified characteristic of the textile subject; and
- producing an output dataset comprising a classification of the textile subject.

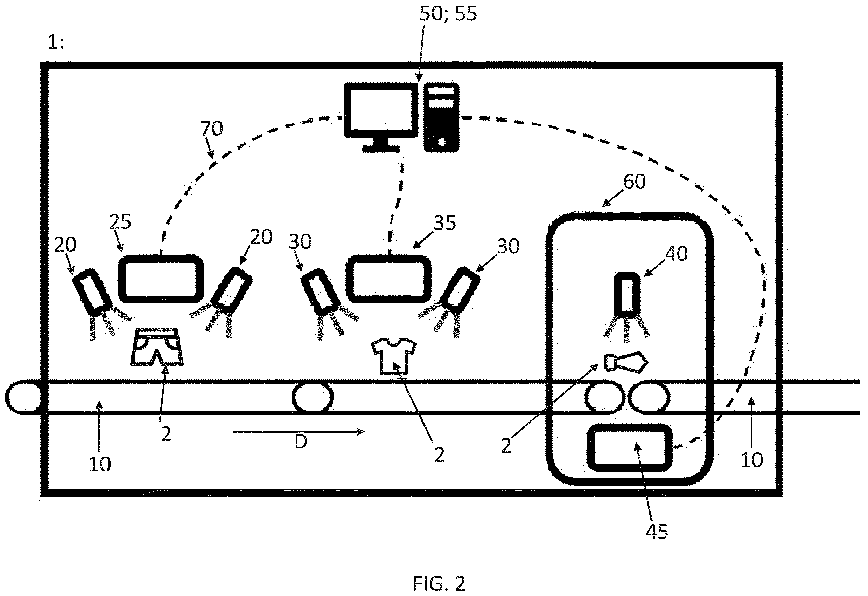

Figure 2 illustrates over the same system as in FIG. 1 except the system comprises a gap in the conveyor, wherein the radiation sensor is positioned underneath the gap of the conveyor.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims may possibly be advantageously combined, and the mentioning of these features in different claims does not exclude that a combination of features is not possible and advantageous.

## Claims

1. A textile identification system (1) for identifying textile subjects (2), wherein said identification system comprises:
- a conveyor (10) for moving an associated textile subject in a first direction (D),
- a visible light emitter (20) for emitting light within a visible wavelength spectrum,
- a colour image sensor (25) for measuring light within a visible wavelength spectrum, preferably a RGB sensor and/or a CMOS sensor and/or CCD sensor,
- an infrared light emitter (30) for emitting light within an infrared wavelength spectrum, preferably within a near infrared wavelength spectrum,
- an infrared light sensor (35) for measuring light within an infrared wavelength spectrum, preferably within a near infrared spectrum,
- a radiation emitter (40) for emitting radiation within an X-ray spectrum or gamma ray spectrum,
- a radiation sensor (45) for measuring radiation within an X-ray spectrum or a gamma spectrum,
- a database system (50) adapted to at least retrieve, store, and process the data measured by at least:
- the colour image sensor (25),
- the infrared light sensor (35), and
- the X-ray or the gamma ray sensor (45).

2. An identification system (1) according to claim 1, wherein the database system (50) is adapted to identify at least one predetermined characteristic of the associated textile subject (2).

3. An identification system (1) according to claim 2, wherein the identification system further comprises an algorithm (55), said algorithm is adapted to provide a classification of the associated textile subject (2) based on the at least one predetermined characteristic.

4. An identification system (1) according to any of the preceding claims, wherein the identification system is adapted to provide a command for sortation of the associated textile subject based on the classification.

5. An identification system (1) according to claim 3, wherein the algorithm is a machine learning algorithm adapted to be trained by the collected data from the database (50).

6. An identification system (1) according to any of the preceding claims, wherein the radiation sensor (45) is positioned underneath the conveyor (10), and wherein the conveyor or part of the conveyor above the radiation sensor is made of material suitable for not blocking X-rays or gamma rays.

7. An identification system (1) according to any preceding claims, wherein the position of the textile subject (2) on the conveyor is measured by at least one of the sensors (25, 35, 45), and wherein a robot, preferably a robot arm, is adapted to collect the subject for sortation.

8. An identification system (1) according to any preceding claims, wherein the system further comprises a shielding (60) for shielding from the radiation of the radiation emitter (40).

9. A method for identification of a textile subject, preferably in an identification system according to claim 1, wherein said method comprises the steps of:
- transporting a textile subject (2) in a first direction (D), preferably on a conveyor (10),
- emitting light at the textile subject by at least one first light emitter (20), said first light emitter emitting light within the visible light spectrum,
- measuring colour distributed over the textile subject by at least one colour image sensor (25), preferably a RGB sensor and/or CMOS sensor and/or CCD sensor,
- emitting infrared light on the subject by at least one infrared light emitter (30),
- measuring reflected light by at least one infrared light sensor (35),
- emitting X-ray or gamma-ray radiation through the textile subject by a radiation emitter (40),
- measuring radiation by at least one radiation sensor (45),
- collecting data measured by the colour image sensor, the infrared light sensor and the radiation sensor,
- identifying at least one or more characteristics of the textile subject (2), preferably identifying at least:
- the colour,
- the material composition, and
- the presence of hard material, such as buttons and zippers, and
- classifying the subject, preferably into predetermined classification groups,
wherein the steps of the method can be executed in any order and/or executed simultaneously.

10. A method for identification according to claim 9, wherein the method further comprises the step of:
- storing the measured and collected data in a database (50).

11. A method for identification according to claim 9-10, wherein the method further comprises the step of:
- providing at least one algorithm (55).

12. A method for identification according to claim 11, wherein the method further comprises the step of:
- using the stored data in the database (50) for machine learning by training the at least one algorithm (55),
said algorithm preferably being trained for at least one of:
- optimizing the identification of the characteristics of the textile subject (2),
- optimizing the classification of the textile subject (2), and/or
- optimizing the sortation of the textile subject (2).

13. A method for identification according to claim 12, wherein the method further comprises the step of:
- using the machine learning algorithm for at least one of:
- identification of characteristics of the textile subject,
- classification of the subject into predetermined classification groups, and/or
- sortation of the subject into predetermined sortation groups.

14. A method for identification according to claim 9-13, wherein the method further comprises the step of:
- choosing predetermined sortation groups,
- sorting the subject based on the one or more predetermined sortation groups, preferably wherein the sortation of the subject is based on the at least one algorithm.

15. An identification system according to claim 1-8 and/or a method according to claim 9-14, wherein the characteristics of the textile subject identified from the measurements of the one or more sensors is one or more of:
- colour distribution, such as colour gradients,
- fabric type, such as weave, knit, terry cloth and/or denim,
- material composition,
- density,
- mass,
- condition, such as cleanness, moisture content and/or mould content,
- presence of reflective material,
- presence of pattern, such as stripes and/or squares,
- presence of polymer prints,
- presence of hard accessories such as buttons, zippers, pens and/or tools,
- presence of contaminant chemicals such as oil,
- presence of multi-layer textile, such as jackets, pillows and duvets,
- type of textile subject, such as trousers, jeans, a shirt, a bag or a shoe, or
- the subject is not a textile subject.

16. A computer program, comprising instructions which, when the program is executed by a computer, cause the computer to carry out at least the identifying and classification step of the method of claim 9, preferably also the sortation step of claim 14.

17. A computer-implemented method of classifying textile subjects, comprising:
- receiving an input dataset, according to the measurement steps of claim 9, the input dataset comprising at least one identified characteristic of the textile subject according to claim 15; and
- producing an output dataset comprising a classification of the textile subject.

## Amended claims

### Amended claims in accordance with Rule 137(2) EPC.

1. A textile identification system (1) for identifying textile subjects (2), wherein said identification system comprises:
- a conveyor (10) for moving an associated textile subject in a first direction (D),
- a vision or optical recognition system,
- an infrared light emitter (30) for emitting light within an infrared wavelength spectrum, preferably within a near infrared wavelength spectrum,
- an infrared light sensor (35) for measuring light within an infrared wavelength spectrum, preferably within a near infrared spectrum,
- a radiation emitter (40) for emitting radiation within an X-ray spectrum or gamma ray spectrum,
- a database system (50) adapted to at least retrieve, store, and process the data measured by at least:
- the colour image sensor (25), and
- the infrared light sensor (35) **characterized in that** the system further comprises
- a visible light emitter (20) for emitting light within a visible wavelength spectrum which visible light emitter (20) has a light spectrum within 300-1000nm, and
- a radiation sensor (45) for measuring radiation within an X-ray spectrum or a gamma spectrum by using absorption spectroscopy such as X-ray or gamma ray absorption spectroscopy, which radiation sensor (45) is positioned underneath the conveyor (10), and wherein the conveyor or part of the conveyor above the radiation sensor (45) is made of material suitable for not blocking X-rays or gamma rays and that
- the vision or optical recognition system comprises a colour image sensor (25) for measuring light within a visible wavelength spectrum, preferably a RGB sensor and/or a CMOS sensor and/or CCD sensor and that
- the database system (50) is adapted to retrieve, store, and process the data measured by the radiation sensor (45)
- wherein the radiation sensor (45) is positioned underneath the conveyor (10), and wherein the conveyor or part of the conveyor above the radiation sensor is made of material suitable for not blocking X-rays or gamma rays.

2. An identification system (1) according to claim 1, wherein the database system (50) is adapted to identify at least one predetermined characteristic of the associated textile subject (2).

3. An identification system (1) according to claim 2, wherein the identification system further comprises an algorithm (55), said algorithm is adapted to provide a classification of the associated textile subject (2) based on the at least one predetermined characteristic.

4. An identification system (1) according to claim 3, wherein the identification system is adapted to provide a command for sortation of the associated textile subject based on the classification.

5. An identification system (1) according to claim 3, wherein the algorithm is a machine learning algorithm adapted to be trained by the collected data from the database (50).

6. An identification system (1) according to any of the preceding claims, wherein the position of the textile subject (2) on the conveyor is measured by at least one of the sensors (25, 35, 45), and wherein the identification system comprises a robot, preferably a robot arm, which is adapted to collect the subject for sortation.

7. An identification system (1) according to any of the preceding claims, wherein the system further comprises a shielding (60) for shielding from the radiation of the radiation emitter (40).

8. A method for identification of a textile subject, preferably in an identification system according to claim 1, wherein said method comprises the steps of:
- transporting a textile subject (2) in a first direction (D), preferably on a conveyor (10),
- recognizing the textile subject using a vision or optical system,
- emitting infrared light on the subject by at least one infrared light emitter (30),
- measuring reflected light by at least one infrared light sensor (35),
- emitting X-ray or gamma-ray radiation through the textile subject by a radiation emitter (40),
- collecting data measured by the colour image sensor and the infrared light sensor,
- identifying at least one or more characteristics of the textile subject (2), and
- classifying the textile subject, preferably into predetermined classification groups,
- **characterized in that** the method further comprises the steps of:
- emitting light at the textile subject by at least one first light emitter (20), said first light emitter emitting light within the visible light spectrum, which visible light emitter (20) has a light spectrum within 300-1000nm,
- providing the vision or optical recognition system in form of a colour image sensor (25) for measuring light within a visible wavelength spectrum, preferably a RGB sensor and/or a CMOS sensor and/or CCD sensor,
- measuring colour distributed over the textile subject by at least one colour image sensor (25), preferably a RGB sensor and/or CMOS sensor and/or CCD sensor,
- providing a radiation sensor (45) in a position underneath the conveyor (10),
- providing the conveyor or part of the conveyor above the radiation sensor (45) of material suitable for not blocking X-rays or gamma rays,
- measuring with the radiation sensor (45) radiation within an X-ray spectrum or a gamma spectrum by using absorption spectroscopy such as X-ray or gamma ray absorption spectroscopy, which radiation sensor (45),
- collecting data measured by the radiation sensor (45),
- storing the measured and collected data in a database (50),
- identifying - based on the collected data - at least one or more characteristics of the textile subject (2), preferably identifying at least:
- the colour,
- the material composition, and
- the presence of hard material, such as buttons and zippers, and in which method
- the steps of the method can be executed in any order and/or executed simultaneously.

9. A method for identification according to claim 8, wherein the method further comprises the step of:
- providing at least one algorithm (55).

10. A method for identification according to claim 9, wherein the method further comprises the step of:
- using the stored data in the database (50) for machine learning by training the at least one algorithm (55),
said algorithm preferably being trained for at least one of:
- optimizing the identification of the characteristics of the textile subject (2),
- optimizing the classification of the textile subject (2), and/or
- optimizing the sortation of the textile subject (2).

11. A method for identification according to claim 10, wherein the method further comprises the step of:
- using the machine learning algorithm for at least one of:
- identification of characteristics of the textile subject,
- classification of the subject into predetermined classification groups, and/or
- sortation of the subject into predetermined sortation groups.

12. A method for identification according any of the claims 8-11, wherein the method further comprises the step of:
- choosing predetermined sortation groups,
- sorting the subject based on the one or more predetermined sortation groups, preferably wherein the sortation of the subject is based on the at least one algorithm.

13. An identification system according to any of the claims 1-7, wherein characteristics of the textile subject identified from the measurements of the one or more sensors is one or more of:
- colour distribution, such as colour gradients,
- fabric type, such as weave, knit, terry cloth and/or denim,
- material composition,
- density,
- mass,
- condition, such as cleanness, moisture content and/or mould content,
- presence of reflective material,
- presence of pattern, such as stripes and/or squares,
- presence of polymer prints,
- presence of hard accessories such as buttons, zippers, pens and/or tools,
- presence of contaminant chemicals such as oil,
- presence of multi-layer textile, such as jackets, pillows and duvets,
- type of textile subject, such as trousers, jeans, a shirt, a bag or a shoe, or
- the subject is not a textile subject.

14. A computer program, comprising instructions which, when the program is executed by a computer, cause the computer to carry out at least the identifying and classification step of the method of claim 8.

15. A computer-implemented method of classifying textile subjects, comprising:
- receiving an input dataset, according to the measurement steps of claim 8, the input dataset comprising at least one identified characteristic of the textile subject according to claim 13; and
- producing an output dataset comprising a classification of the textile subject.
